(19) Europäisches Patentamt
European Patent Office
Office européen des brevets

(11) Veröffentlichungsnummer : **0 014 443**
**B1**

(12) # EUROPÄISCHE PATENTSCHRIFT

(45) Veröffentlichungstag der Patentschrift : **03.11.82**

(51) Int. Cl.³ : **C 07 D235/32**, A 01 N 43/52

(21) Anmeldenummer : **80100507.5**

(22) Anmeldetag : **01.02.80**

(54) **Verdünnbare Benzimidazolcarbaminsäurealkylester-Formulierung, Verfahren zu ihrer Herstellung und ihre Verwendung als Biozid.**

(30) Priorität : **06.02.79 DE 2904390**

(43) Veröffentlichungstag der Anmeldung : **20.08.80 (Patentblatt 80/17)**

(45) Bekanntmachung des Hinweises auf die Patenterteilung : **03.11.82 Patentblatt 82/44**

(84) Benannte Vertragsstaaten : **AT CH DE FR GB NL SE**

(56) Entgegenhaltungen : **DE A 2 230 182**

(73) Patentinhaber : **RIEDEL-DE HAEN AKTIENGESELLSCHAFT**
**Wunstorfer Strasse 40**
**D-3016 Seelze 1 (DE)**

(72) Erfinder : **Gattner, Hans, Dr.**
**Harrenhorst 15**
**D-3052 Bad Nenndorf (DE)**
Erfinder : **Wagner, Karl, Dr.**
**Lindenstrasse 7**
**D-3016 Seelze (DE)**

(74) Vertreter : **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

**EP 0 014 443 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verdünnbare Benzimidazolcarbaminsäurealkylester-Formulierung, Verfahren zu ihrer Herstellung und ihre Verwendung als Biozid

Die Erfindung betrifft eine verdünnbare Benzimidazolcarbaminsäurealkylester-Formulierung, ein Verfahren zu ihrer Herstellung und ihre Verwendung als Biozid.

Benzimidazolcarbaminsäurealkylester, im folgenden mit BCAE bezeichnet, sind als Fungizide für den Pflanzenschutz und für den Schutz technischer Materialien gegen mikrobiellen Abbau bekannt.

Ihre Anwendung wird jedoch stark durch den Umstand eingeschränkt, daß sie in Wasser und in den üblichen Lösungsmitteln praktisch unlöslich sind. Um diesen Nachteil zu beseitigen, wurde vorgeschlagen, BCAE zur Bekämpfung von Pilzbefall an lebenden Pflanzen in sehr fein verteilter Form, d.h. in einer Teilchengröße von 5 µm, einzusetzen (DE-OS 16 20 175), weil Teilchen dieser Größenordnung auf der Oberfläche von Pflanzenteilen haften. Ein Eindringen fester Teilchen in die Oberfläche von zu behandelnden Materialien ist jedoch nicht zu erwarten, so daß der festgestellte Nachteil durch die vorgeschlagene Maßnahme nicht beseitigt wird.

Es bestand daher die Aufgabe, eine BCAE enthaltende Formulierung bereitzustellen, die verdünnbar ist.

Zur Lösung dieser Aufgabe wurde versucht, BCAE durch Einbau von Substituenten Löslich zu machen, z.B. durch Umsetzung mit Isocyanat zu 1-Carbamoyl-BCAE-Derivaten (DE-OS 17 45 784). Diese Carbamoyl-Derivate haben zwar ein ähnlich günstiges Wirkungsspektrum wie BCAE, unterscheiden sich aber auch im Löseverhalten nur geringfügig von diesen.

Andere Derivate sind die 1-Aminomethyl-BCAE-Derivate (DE-OS 22 30 182), die jedoch in Lösemitteln keine stabile Lösungen ergeben, da sie zur Dissoziation in die Ausgangskomponenten neigen.

Gleiches gilt für die 2-Triazino-Benzimidazole, die durch Reaktion von BCAE mit einem primären Amin und der doppelt molaren Menge Formaldehyd entstehen (DE-OS 22 24 244).

In polaren Lösungsmitteln wesentlich besser löslich sind Salze von BCAE mit starken Säuren (DE-OS 17 92 688). Diese Produkte werden deswegen auch im Holzschutz als Mittel gegen holzverblauende Pilze eingesetzt. Sie haben jedoch den Nachteil, daß sie in Formulierungen, die unpolare Lösungsmittel wie z.B. Testbenzin enthalten, zum Ausfällen der Wirkstoffe neigen. Weiterhin sind sie sehr anfällig gegenüber Hydrolyse. Bereits geringe Spuren von Wasser führen zur Ausfällung des schwer löslichen BCAE-Körpers.

Die Erfindung betrifft nun eine verdünnbare BCAE-Formulierung, die durch Umsetzung eines Benzimidazolcarbaminsäurealkylesters (BCAE) der allgemeinen Formel (I)

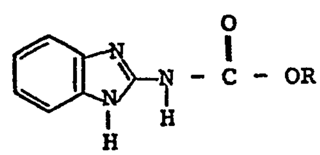

(I)

worin R eine niedere Alkylgruppe bedeutet, mit mindestens der doppelt molaren Menge eines aliphatischen Mono- oder Dialdehyds mit 1 bis 10 Kohlenstoffatomen oder Benzaldehyd in Gegenwart mindestens der doppelt molaren Menge eines primären, sekundären oder tertiären Alkohols mit 1 bis 18 Kohlenstoffatomen erhältlich ist. Das Symbol R bedeutet vorzugsweise eine Alkylgruppe mit 1 bis 4 Kohlenstoffatomen.

Die erfindungsgemäßen BCAE-Formulierungen stellen Produkte dar, die in Lösung stabil sind. Sie weisen darüberhinaus weitere Vorteile auf :

a) Die erfindungsgemäßen BCAE-Formulierungen sind gut verträglich mit Holzschutzmittelformulierungen auf Basis unpolarer Lösungsmittel, z.B. Testbenzin. Zwar können auch Verbindungen gemäß Stand der Technik, z.B. BCAE-n-dodecylbenzolsulfonat, in Äthylglykol gelöst und Holzschutzmittelformulierungen auf Basis unpolarer Lösungsmittel zugesetzt werden, jedoch scheidet sich häufig im Verlauf einiger Wochen ein Bodensatz von BCAE ab, wodurch die Wirkung des Holzschutzmittels gegen Bläuepilze beeinträchtigt wird.

b) Ferner weisen Holzschutzmittelformulierungen, die die erfindungsgemäßen BCAE-Formulierungen enthalten, Eindringtiefen in Holz von mehreren Millimetern auf, die mit BCAE-Verbindungen gemäß Stand der Technik nicht erreicht werden.

c) Außerdem lassen sich mit den erfindungsgemäßen BCAE-Formulierungen kombinierte Holzschutzmittel in Lösungsform herstellen, die sowohl gegen holzzerstörende Pilze und holzzerstörende Insekten als auch gegen Bläuepilze wirken, z.B. ein flüssiges Wirkstoffkonzentrat mit z.B. Tributylzinnoxid gegen holzzerstörende Pilze und z.B. Endosulfan gegen holzzerstörende Insekten. Das ist mit den BCAE-Verbindungen gemäß Stand der Technik nicht möglich.

Benzimidazolcarbaminsäurealkylester stehen als Handelsprodukte zur Verfügung. Sie sind herstellbar z.B. nach dem Cyanamid-Verfahren (DE-OS 16 68 557). Für die Erfindung eignen sich vorzugsweise

BCAE-Verbindungen mit 1 bis 4 Kohlenstoffatomen in der Alkylgruppe. Insbesondere kommt der Benzimidazolcarbaminsäuremethylester (BCM) in Betracht.

Als Aldehyde mit 1 bis 10 Kohlenstoffatomen eignen sich besonders aliphatische Mono- oder Dialdehyde mit vorzugsweise 1 bis 5 Kohlenstoffatomen, z.B. Formaldehyd, Paraformaldehyd, Acetaldehyd, Propionaldehyd, Glutardialdehyd, Glyoxal, Glyoxylsäure und Acrolein, sowie Benzaldehyd.

Als Alkohole kommen Alkanole mit 1 bis 18 Kohlenstoffatomen, vorzugsweise 1 bis 10 Kohlenstoffatomen, Glykole, Glykoläther und polyalkoxylierte Alkylphenole in Betracht. Geeignet sind insbesondere geradkettige oder verzweigte, primäre, sekundäre oder tertiäre Alkohole mit 1 bis 18 Kohlenstoffatomen, z.B. Methanol, Äthanol, Propanol, Isopropanol, Butanol, Isobutanol, 3-Methylbutanol, 2-Äthylhexanol, Isoamylalkohol, Isodecylalkohol, Isotridecylalkohol, Isooctadecylalkohol, 3-Methoxybutanol, 4-Methyl-4-hydroxy-pentanon-(2) und Glykolsäure-n-butylester, ferner Glykole, z.B. Äthylenglykol, Glycerin, Diäthylenglykol und Triäthylenglykol, sowie Glykoläther, z.B. Methylglykol, Äthylglykol, Butylglykol, Methyldiglykol, Äthyldiglykol und Butyldiglykol, und auch polyalkoxylierte Produkte aus alkylierten Phenolen und Äthylenoxid oder Propylenoxid, z.B. das Umsetzungsprodukt aus Nonylphenol und 10 Äquivalenten Äthylenoxid.

Die Auswahl der verwendeten hydroxylgruppenhaltigen Verbindung richtet sich nach dem vorgesehenen Anwendungszweck. Formulierungen, die in unpolare Lösemittel, z.B. Benzin, enthaltenden Systemen eingesetzt werden sollen, werden vorteilhalft auf Basis von Butylglykol hergestellt. Für Formulierungen, die wasserlöslich sein sollen, sind als hydroxylgruppenhaltige Verbindungen Methylglykol und Äthylglykol besser geeignet. Auch Polyglykoläther-Verbindungen lassen sich mit Vorteil verwenden. So ist die Formulierung aus BCAE, Formaldehyd und einem handelsüblichen Umsetzungsprodukt aus Nonylphenol und 10 Mol Äthylenoxid sehr gut geeignet, um wässrige Dispersionen herzustellen.

Bevorzugt wird nur ein Aldehyd und nur ein Alkohol aus den angegebenen Gruppen verwendet. Man kann aber auch Gemische von zwei Aldehyden oder aus zwei Alkohole verwenden. Vorteilhaft wird ein größerer Überschuß des Aldehyds und/oder des Alkohols angewendet.

Bevorzugt sind daher Formulierungen, bei denen der Anteil der Verbindung (I) 10 bis 20 Gewichtsprozent des gesamten Reaktionsgemisches beträgt.

Bei der Herstellung der erfindungsgemäßen Formulierungen ist zu beachten, daß diese möglichst rasch und bei nicht zu hohen Temperaturen vorgenommen werden soll. Andernfalls leidet die Wirksamkeit.

Die erfindungsgemäßen Formulierungen lassen sich nach einem Verfahren herstellen, bei dem BCAE der allgemeinen Formel (I) in mindestens der doppelt molaren Menge mindestens eines Alkohols suspendiert, unter ständigem Bewegen bei 40 bis 150 °C, bevorzugt 80 bis 110 °C, mit mindestens der doppelt molaren Menge mindestens eines Aldehyds mit 1 bis 10 Kohlenstoffatomen versetzt und bis zur vollständigen Auflösung bei dieser Temperatur gehalten wird. Das Bewegen und Durchmischen der Reaktionspartner wird vorzugsweise durch Rühren oder auch durch Schütteln durchgeführt.

Das verwendete BCAE sollte zur Erzielung einer kurzen Umsetzungszeit möglichst feinteilig sein. Vorteilhaft werden Produkte mit durchschnittlichen Korngrößen von 2 bis 10 µm verwendet. Gasförmige oder stark gasende flüssige Reaktionspartner können mit einem Tauchrohr in die Reaktionsmischung eingeleitet werden.

Die erfindungsgemäßen Formulierungen werden als Biozide im Pflanzenschutz und zum Schutz von technischen Materialien gegen mikrobiellen Abbau verwendet. Sie werden direkt oder in lösemittelhaltigen oder wässrigen Pflanzenschutzmitteln oder Konservierungsmitteln eingesetzt. Insbesondere eignen sie sich zur Vorbeugung oder Behandlung von Schimmelpilzfall auf Textilien, Leder und Papier und in Anstrichmitteln und anderen Beschichtungsmitteln.

Für diese Einsatzzwecke können sie in Kombination mit anderen Fungiziden und mit Bakteriziden oder Algiziden verwendet werden. Dafür kommen z.B. in Frage : zinnorganische Verbindungen, Dithiocarbamate, Isothiazolone, Benzisothiazol-3-on, Hexahydrotriazine, Chloracetamid, N-Trichlormethylthiotetrahydrophthalimid (Captan), n-Trichlormethylthiophthalimid (Folpet), Fluorfolpet, 3-(3',4'-Dichlorphenyl)-1,1-dimethylharnstoff (Diuron), Pentachlornitrobenzol (Folsan), 1-n-Butyl-aminocarbonyl-2-methoxycarbonylaminobenzimidazol und N-Dichlorfluormethylthio-N',N'-dimethylaminosulfonsäureanilid.

Als Anwendungsmenge kommen 0,001 bis 15 Gewichtsprozent, vorzugsweise 0,3 bis 5 Gewichtsprozent der erfindungsgemäßen Formulierung, gegebenenfalls im Gemisch mit anderen Wirkstoffen (bezogen auf die Menge des biozid auszurüstenden Anstrich- oder Beschichtungsmittels), in Betracht.

Die Erfindung wird durch die folgenden Beispiele näher erläutert. Die Abkürzung « GT » bedeutet « Gewichtsteile ». Prozentangaben beziehen sich auf das Gewicht.

## Beispiel 1

100 g (0,524 Mol) Benzimidazolcarbaminsäuremethylester (BCM) und
200 g (6,667 Mol) Paraformaldehyd werden unter Rühren in
700 g (5,932 Mol) Butylglykol eingetragen und auf 100 °C erwärmt. Nach etwa 1 Stunde ist eine klare Lösung entstanden.

Beispiel 2

200 g (1,047 Mol) BCM und
200 g (6,667 Mol) Paraformaldehyd werden unter Rühren in
600 g (5,085 Mol) Butylglykol eingetragen und auf 100 °C erwärmt. Nach etwa 1 Stunde haben sich die Feststoffe gelöst.

Beispiel 3

200 g ( 1,047 Mol) BCM und
320 g (10,667 Mol) Paraformaldehyd werden unter Rühren in
480 g ( 4,068 Mol) Butylglykol eingetragen und auf 90 °C erwärmt. Nach etwa 1 Stunde hat sich alles gelöst.

Beispiel 4

200 g (1,047 Mol) BCM und
240 g (8,000 Mol) Paraformaldehyd werden unter Rühren in
560 g (4,756 Mol) Butylglykol eingetragen und auf 105 °C erwärmt. Nach 50 Minuten sind die Feststoffe in Lösung gegangen.

Die nach Beispiel 1 bis 4 erhaltenen Formulierungen lassen sich jeweils mit 500 g Testbenzin verdünnen, ohne daß eine Ausfällung eintritt.

Beispiel 5

154 kg eines nutschenfeuchten Produktes aus einem technischen Ansatz, entsprechend
100,1 kg (0,524 kmol) BCM und
50,05 kg (0,833 kmol) Isopropanol und
3,85 kg (0,214 kmol) Wasser werden unter Rühren in
646 kg (5,475 kmol) Butylglykol eingetragen. Nach Zusatz von
200 kg (6,667 kmol) Paraformaldehyd wird unter Rühren auf 100 °C erwärmt. Nach Verlauf einer Stunde hat sich alles gelöst.

Beispiel 6

100 g (0,524 Mol) BCM und
200 g (6,667 Mol) Paraformaldehyd werden unter Rühren in
540 g (6,000 Mol) Äthylglykol eingetragen und auf 100 °C erwärmt. Nach 70 Minuten ist Lösung eingetreten.

Beispiel 7

100 g (0,524 Mol) BCM und
200 g (6,667 Mol) Paraformaldehyd werden unter Rühren in
350 g (5,645 Mol) Äthylenglykol eingetragen und auf 110 °C erwärmt. Nach etwa 1 Stunde haben sich die Feststoffe gelöst.

Beispiel 8

100 g (0,524 Mol) BCM und
200 g (6,667 Mol) Paraformaldehyd werden unter Rühren in
600 g (5,521 Mol) Glycerin eingetragen und auf 110 °C erwärmt. Nach etwa 80 Minuten ist alles gelöst.

Beispiel 9

100 g (0,524 Mol) BCM und
200 g (6,667 Mol) Paraformaldehyd werden unter Rühren in
400 g (5,405 Mol) n-Butanol eingetragen und auf 105 °C erwärmt. Nach 70 Minuten hat sich alles gelöst.

Die nach Beispiel 6 bis 9 erhaltenen Formulierungen können mit jeweils 500 g Wasser verdünnt werden, ohne daß eine Ausfällung eintritt.

Zum Vergleich : Die Löslichkeit von BCM beträgt in Wasser 6 ppm und in Butanol 600 ppm.

In gleicher Weise werden folgende Formulierungen hergestellt :

Tabelle 1

| Beispiel Nr. | Reaktanden | g | mol | °C | Minuten |
|---|---|---|---|---|---|
| 10 | BCM | 100 | 0,524 | 100 | 60 |
| | Paraformaldehyd | 200 | 6,667 | | |
| | Triäthylenglykol | 700 | 4,667 | | |
| 11 | BCM | 100 | 0,524 | 95 | 70 |
| | Glyoxylsäure-1-hydrat (97 %ig) | 250 | 2,636 | | |
| | Butylglykol | 250 | 2,119 | | |
| 12 | BCM | 100 | 0,524 | 95 | 60 |
| | Glyoxylsäure-1-hydrat (97 %ig) | 250 | 2,636 | | |
| | Diäthylenglykol | 200 | 1,887 | | |
| 13 | BCM | 100 | 0,524 | 100 | 60 |
| | Glutardialdehyd (50 %ig in $H_2O$) | 400 | 2,000 | | |
| | 3-Methoxybutanol | 400 | 3,846 | | |
| 14 | BCM | 100 | 0,524 | 100 | 60 |
| | Glutardialdehyd (50 %ig in $H_2O$) | 400 | 2,000 | | |
| | Glykolsäure-n-butylester | 400 | 3,030 | | |
| 15 | Benzimidazolcarbaminsäureäthylester | 100 | 0,488 | 100 | 70 |
| | Paraformaldehyd | 200 | 6,667 | | |
| | Äthylglykol | 700 | 7,778 | | |
| 16 | Benzimidazolcarbaminsäurepropylester | 100 | 0,457 | 100 | 70 |
| | Paraformaldehyd | 200 | 6,667 | | |
| | Butylglykol | 700 | 5,932 | | |

## Beispiel 17

100 g (0,524 Mol) BCM werden in
200 g (2,703 Mol) Butanol suspendiert und auf 80 °C erwärmt. Unter Rühren werden innerhalb 1 Stunde
200 g (3,448 Mol) Propionaldehyd durch ein Tauchrohr eingeleitet. Zur vollständigen Lösung wird noch 20 Minuten auf 100 °C erwärmt.

## Beispiel 18

100 g (0,524 Mol) BCM werden in
300 g (2,542 Mol) Butylglykol suspendiert und auf 80 °C erwärmt. Unter Rühren werden innerhalb 1 Stunde
200 g (3,448 Mol) Propionaldehyd durch ein Tauchrohr eingeleitet. Nach beendeter Einleitung wird 20 Minuten auf 100 °C erwärmt.

## Beispiel 19

200 g (1,047 Mol) BCM werden unter Rühren in
300 g (6,522 Mol) Äthanol suspendiert und auf 75 °C erwärmt.
150 g (2,545 Mol) Acrolein, 95 %ig, werden in 80 Minuten durch ein Tauchrohr eingeleitet. Nach weiteren 20 Minuten Rühren bei 75 °C und Abkühlen wird die leicht trübe Lösung filtriert. Der stechende Geruch des Acrolein ist vollständig verschwunden.

## Beispiel 20

200 g (1,047 Mol) BCM werden unter Rühren in
300 g (4,054 Mol) Butanol suspendiert und auf 80 °C erwärmt.
150 g (2,545 Mol) Acrolein, 95 %ig, werden in 80 Minuten durch ein Tauchrohr eingeleitet. Die leicht trübe Reaktionslösung wird nach dem Abkühlen filtriert. Der stechende Geruch des Acrolein ist vollständig verschwunden.

Beispiel 21

200 g (1,047 Mol) BCM werden unter Rühren in
300 g (3,333 Mol) Äthylglykol suspendiert und auf 80 °C erwärmt.
150 g (2, 545 Mol) Acrolein, 95 %ig, werden in 80 Minuten durch ein Tauchrohr eingeleitet. Die leicht trübe Reaktionslösung wird nach dem Abkühlen filtriert. Der stechende Geruch des Acrolein ist vollständig verschwunden.

Beispiel 22

150 g (5,000 Mol) Paraformaldehyd werden unter Rühren in
750 g (1,136 Mol) des Umsetzungsproduktes von Nonylphenol mit 10 Äquivalenten Äthylenoxid suspendiert und auf 100 °C erwärmt. Dabei löst sich Paraformaldehyd praktisch vollständig auf.
100 g (0,524 Mol) BCM werden eingetragen und bei 100 °C in 80 Minuten gelöst.

Beispiel 23

Die fungizide Wirksamkeit der erfindungsgemäßen Mittel wird nach bekannten Verfahren im Nährbodenplattentest geprüft. Verwendet wird ein Pilznährboden aus 3 % Malzextrakt, 3 % Agar-Agar und 94 % Wasser. Je 20 ml Nährboden werden mit abgestuften Mengen der Testmischungen versetzt und unter sterilen Bedingungen in Standard-Petrischalen von 9 cm Durchmesser gefüllt. Beimpft wird mit Sporensuspensionen von Schrägrohrkulturen. Jede Petrischale wird mit ca. 100 000 Keimen des jeweiligen Testorganismus versetzt. Bebrütet wird 2 Wochen lang bei einer optimalen Wuchstemperatur von 28 °C und 75 % relativer Luftfeuchtigkeit.

In der Tabelle 2 sind die minimalen Hemmkonzentrationen (MHK-Werte), bezogen auf Gewicht der Nährböden, aufgeführt. Bei diesen Konzentrationen wird das Pilzwachstum vollständig gehemmt. Bei der nächstniedrigeren Konzentration tritt zwar noch deutliche Hemmwirkung, jedoch in der Regel bereits innerhalb einer Woche Flächenwachstum der Testpilze auf.

Für den Vergleich der Wirksamkeit gegen in der Praxis des Bläueschutzes wirksame Pilze werden folgende Substanzen eingesetzt :

I Produkt gemäß Beispiel 5
II Benzimidazolcarbaminsäuremethylester (als Vergleich)
III Benzimidazolcarbaminsäuremethylester-n-dodecylbenzolsulfonat (als Vergleich).

Tabelle 2

| Testpilz | MHK-Werte (%) I* | II | III |
|---|---|---|---|
| Aspergillus niger | 0,002 | 0,000 5 | 0,000 5 |
| Aureobasidium pullulans | 0,000 5 | 0,000 05 | 0,000 05 |
| Chaetomium globosum | 0,000 5 | 0,000 05 | 0,000 05 |
| Penicillium funiculosum | 0,000 5 | 0,000 05 | 0,000 05 |

* Beim Vergleich der Wirksamkeiten ist zu berücksichtigen, daß das Produkt gemäß Beispiel 5 nur 10 % BCM enthält, während beim Test gleiche Mengen der Produkte I, II und III eingesetzt werden.

Beispiel 24

25 GT eines Produktes gemäß Beispiel 1 werden zu 975 GT einer üblichen Holzschutzmittelformulierung gegeben, die zusammengesetzt ist aus 140 GT eines handelsüblichen Alkyldharzes, 4 GT Zinknaphthenat als Trockner, 4 GT eines handelsüblichen Hautverhütungsmittels, 30 GT Butyldiglykol und 797 GT Testbenzin 60.

Die Wirksamkeit der so biozid ausgerüsteten Holzschutzmittelformulierung gegen holzverblauende Pilze wird nach der Mündener Streifenmethode [H. Butin, Farbe und Lack, *71* (1965), 373] geprüft. Es ergibt sich eine bewuchsfreie Eindringtiefe von 3 bis 4 mm.

Zum Vergleich wird die oben angegebene übliche Holzschutzmittelformulierung mit 0,25 % Benzimidazolcarbaminsäuremethylester versetzt und unter gleichen Bedingungen geprüft. Es werden nur Eindringtiefen von 0,5 mm festgestellt.

Beispiel 25

50 GT eines nach Beispiel 5 hergestellten Produktes werden mit 23 GT Butylglykol, 25 GT Tributylzinnoxid und 2 GT Endosulfan (α,β,1,2,3,4,7,7-Hexachlorbicyclo-(2,2,1)-hepten-(2)-bisoxomethylen-(5,6)-sulfit) versetzt. Es entsteht eine klare homogene Lösung, die über einen Zeitraum von mindestens 10 Wochen stabil ist.

5 GT einer derartigen Wirkstoff-Formulierung werden zu 95 GT einer handelsüblichen unpigmentierten Holzschutzmittelformulierung gegeben, wie sie in Beispiel 24 beschrieben ist, ohne daß Trübungen oder Ausfällungen entstehen. Auch ein derartiges gebrauchsfertiges kombiniertes Holzschutzmittel erweist sich über einen Zeitraum von mindestens 10 Wochen stabil.

**Ansprüche** (für die Vertragsstaaten : CH, DE, FR, GB, NL, SE)

1. Verdünnbare Benzimidazolcarbaminsäurealkylester-Formulierung, erhältlich durch Umsetzung eines Benzimidazolcarbaminsäurealkylesters der allgemeinen Formel (I)

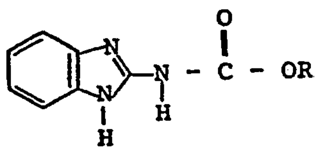

(I)

in der R eine niedere Alkylgruppe bedeutet, mit mindestens der doppelt molaren Menge eines aliphatischen Mono- oder Dialdehyds mit 1 bis 10 Kohlenstoffatomen oder Benzaldehyd in Gegenwart mindestens der doppelt molaren Menge eines primären, sekundären oder tertiären Alkohols mit 1 bis 18 Kohlenstoffatomen.

2. Formulierung nach Anspruch 1, dadurch gekennzeichnet, daß der Anteil der Verbindung der Formel (I) 10 bis 20 Gewichtsprozent des gesamten Reaktionsgemisches beträgt.

3. Verfahren zur Herstellung einer verdünnbaren Benzimidazolcarbaminsäurealkylester-Formulierung, dadurch gekennzeichnet, daß Benzimidazolcarbaminsäurealkylester der allgemeinen Formel (I) in mindestens der doppelt molaren Menge mindestens eines primären, sekundären oder tertiären Alkohols mit 1 bis 18 Kohlenstoffatomen suspendiert, unter ständigem Bewegen bei 40 bis 150 °C mit mindestens der doppelt molaren Menge eines aliphatischen Mono- oder Dialdehyds mit 1 bis 10 Kohlenstoffatomen oder Benzaldehyd versetzt und bis zur vollständigen Lösung bei dieser Temperatur gehalten wird.

4. Verfahren nach Anspruch 3, dadurch gekennzeichnet, daß die Verbindung der allgemeinen Formel (I) in einer Menge eingesetzt wird, die 10 bis 20 Gewichtsprozent des gesamten Reaktionsgemisches beträgt.

5. Verwendung der Formulierung nach Anspruch 1 als Biozid im Pflanzenschutz und als Mittel zum Schutz von technischen Materialien gegen mikrobiellen Abbau.

6. Verwendung nach Anspruch 5 in Kombination mit anderen Fungiziden, Bakteriziden oder Algiziden.

**Ansprüche** (für den Vertragsstaat AT)

1. Verfahren zur Herstellung einer verdünnbaren Benzimidazolcarbaminsäurealkylester-Formulierung, dadurch gekennzeichnet, daß ein Benzimidazolcarbaminsäurealkylester der Formel (I)

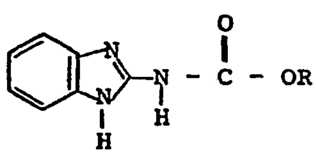

(I)

in der R eine niedere Alkylgruppe bedeutet, in mindestens der doppelt molaren Menge mindestens eines primären, sekundären oder tertiären Alkohols mit 1 bis 18 Kohlenstoffatomen suspendiert, unter ständigem Bewegen bei einer Temperatur von 40 bis 150 °C mit mindestens der doppelt molaren Menge mindestens eines aliphatischen Mono- oder Dialdehyds mit 1 bis 10 Kohlenstoffatomen oder Benzaldehyd versetzt und bis zur vollständigen Lösung bei dieser Temperatur gehalten wird.

2. Verfahren nach Anspruch 1, dadurch gekennzeichnet, daß die Verbindung der Formel (I) in einer Menge von 10 bis 20 Gewichtsprozent des gesamten Reaktionsgemisches eingesetzt wird.

3. Verfahren nach Anspruch 1, dadurch gekennzeichnet, daß die Reaktion bei einer Temperatur von 80 bis 110 °C durchgeführt wird.

4. Verfahren nach Anspruch 1, dadurch gekennzeichnet, daß die Verbindung der Formel (I) in

möglichst feinteiliger Form eingesetzt wird.

5. Verfahren nach Anspruch 4, dadurch gekennzeichnet, daß die Verbindung der Formel (I) in Form von Teilchen mit einem durchschnittlichen Durchmesser von 2 bis 10 μm eingesetzt wird.

6. Verwendung der nach Anspruch 1 hergestellten Formulierung als Biozid im Pflanzenschutz und als Mittel zum Schutz von technischen Materialien gegen mikrobiellen Abbau.

7. Verwendung der nach Anspruch 1 hergestellten Formulierung gemäß Anspruch 6 in Kombination mit anderen Fungiziden, Bakteriziden oder Algiziden.

**Claims** (for the Contracting States : CH, DE, FR, GB, NL, SE)

1. Dilutable benzimidazole carbamic acid alkyl ester formulation, obtainable by chemical reaction of a benzimidazole carbamic acid alkyl ester of the general formula (I)

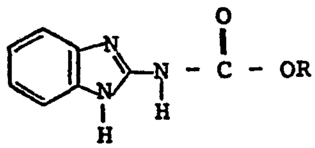
(I)

wherein R means a lower alkyl group with at least the twice molar amount of an aliphatic mono- or dialdehyde having 1 to 10 carbon atoms or benzaldehyde in the presence of at least the twice molar amount of a primary, secondary or tertiary alcohol having 1 to 18 carbon atoms.

2. Formulation according to claim 1, characterised in that the portion of the compound of formula (I) amounts to 10 to 20 weight percent of the total reaction mixture.

3. Process for the manufacture of a dilutable benzimidazole carbamic acid alkyl ester formulation, characterised in that benzimidazole carbamic acid alkyl ester of the general formula (I) is suspended in at least the twice molar amount of a primary, secondary or tertiary alcohol having 1 to 18 carbon atoms, is treated with at least the twice molar amount of an aliphatic mono- or dialdehyde having 1 to 10 carbon atoms or benzaldehyde under permanent agitation at 40 to 150 °C, and is kept at this temperature to complete dissolution.

4. Process according to claim 3, characterised in that the compound of the general formula (I) is used in an amount which is 10 to 20 weight percent of the total reaction mixture.

5. Use of the formulation according to claim 1 as biocide in plant protection and as agent for the protection of technical materials against microbial decomposition.

6. Use according to claim 5 in combination with other fungicides, bactericides or algicides.

**Claims** (for the Contracting State AT)

1. Process for the manufacture of a dilutable benzimidazole carbamic acid alkyl ester formulation, characterised in that a benzimidazole carbamic acid alkyl ester of the formula (I)

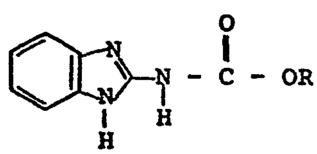
(I)

wherein R means a lower alkyl group is suspended in at least the twice molar amount of a primary, secondary or tertiary alcohol having 1 to 18 carbon atoms, is treated with at least the twice molar amount of an aliphatic mono- or dialdehyde having 1 to 10 carbon atoms or benzaldehyde under permanent agitation at 40 to 150 °C, and is kept at this temperature to complete dissolution.

2. Process according to claim 1, characterised in that the compound of formula (I) is used in an amount of from 10 to 20 weight percent of the total reaction mixture.

3. Process according to claim 1, characterised in that the reaction is carried out at a temperature of from 80 to 110 °C.

4. Process according to claim 1, characterised in that the compound of formula (I) is used in a shape as finely divided as possible.

5. Process according to claim 4, characterised in that the compound of formula (I) is used in the shape of particles having an average diameter of from 2 to 10 micrometers.

6. Use of the formulation manufactured in accordance with claim 1 as biocide in plant protection and as agent for the protection of technical materials against microbial decomposition.

7. Use of the formulation manufactured in accordance with claim 1, according to claim 6, in combination with other fungicides, bactericides or algicides.

**Revendications** (pour les Etats contractants : CH, DE, FR, GB, NL, SE)

1. Formulation diluable à base d'un benzimidazolyl-carbamate d'alkyle que l'on obtient en faisant réagir un benzimidazolyl-carbamate d'alkyle répondant à la formule générale I

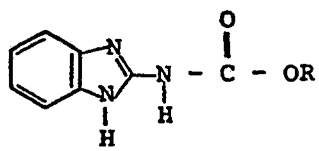

(I)

dans laquelle R représente un radical alkyle inférieur, avec au moins le double de la quantité molaire d'un mono- ou dialdéhyde aliphatique contenant de 1 à 10 atomes de carbone ou de benzaldéhyde, en présence d'au moins le double de la quantité molaire d'un alcool primaire, secondaire ou tertiaire contenant de 1 à 18 atomes de carbone.

2. Formulation selon la revendication 1, caractérisée en ce que la proportion du composé de formule I est comprise entre 10 et 20 % en poids par rapport à l'ensemble du mélange réactionnel.

3. Procédé de préparation d'une formulation diluable à base d'un benzimidazolyl-carbamate d'alkyle, procédé caractérisé en ce qu'on met un benzimidazolyl-carbamate d'alkyle de formule générale I en suspension dans au moins le double de la quantité molaire d'au moins un alcool primaire, secondaire ou tertiaire contenant de 1 à 8 atomes de carbone, on ajoute tout en remuant constamment, à une température de 40 à 150 °C, au moins le double de la quantité molaire d'un mono- ou dialdéhyde aliphatique contenant de 1 à 10 atomes de carbone ou de benzaldéhyde, et on maintient à cette température jusqu'à ce que la dissolution soit complète.

4. Procédé selon la revendication 3, caractérisé en ce que le composé de formule générale I est utilisé en une quantité qui représente de 10 à 20 % en poids du mélange réactionnel total.

5. Application de la formulation selon la revendication 1 comme biocide pour la protection des plantes ou comme agent pour la protection de matières techniques contre les dégradations microbiennes.

6. Application selon la revendication 5 en association avec d'autres fongicides, des bactéricides ou des algicides.

**Revendications** (pour l'Etat contractant AT)

1. Procédé de préparation d'une formulation diluable à base d'un benzimidazolyl-carbamate d'alkyle, procédé caractérisé en ce qu'on met un benzimidazolyl-carbamate d'alkyle répondant à la formule I

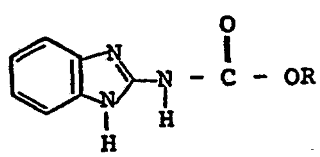

(I)

dans laquelle R représente un radical alkyle inférieur, en suspension dans au moins le double de la quantité molaire d'au moins un alcool primaire, secondaire ou tertiaire contenant de 1 à 18 atomes de carbone, on ajoute, tout en remuant constamment, à une température de 40 à 150 °C, au moins le double de la quantité molaire d'au moins un mono- ou dialdéhyde aliphatique contenant de 1 à 10 atomes de carbone ou de benzaldéhyde, et on maintient à cette température jusqu'à ce que la dissolution soit complète.

2. Procédé selon la revendication 1, caractérisé en ce que le composé de formule I est utilisé en une quantité comprise entre 10 et 20 % en poids par rapport au mélange réactionnel total.

3. Procédé selon la revendication 1, caractérisé en ce que la réaction est effectuée à une température de 80 à 110 °C.

4. Procédé selon la revendication 1, caractérisé en ce que le composé de formule I est mis en jeu sous une forme aussi finement divisée que possible.

5. Procédé selon la revendication 4, caractérisé en ce que le composé de formule I est mis en jeu sous la forme de particules ayant un diamètre moyen de 2 à 10 μm.

6. Application de la formulation préparée selon la revendication 1 comme biocide pour la protection des plantes et comme agent pour la protection de matières techniques contre les dégradations microbiennes.

7. Application, selon la revendication 6, de la formulation préparée selon la revendication 1, en association avec d'autres fongicides, des bactéricides ou des algicides.